# EUROPEAN PATENT APPLICATION

(11) **EP 4 782 002 A1**
(43) Date of publication of application: **29.07.2026**
(21) Application number: 25382047.6
(22) Date of filing: 27.01.2025
(51) Int. Cl.: A61K 38/44, A61K 31/519, A61P 15/08

(54) **DIAMINE OXIDASE (DAO) FOR USE IN THE TREATMENT OF INFERTILITY**

(71) Applicant: DR Healthcare S.L.U., 08017 Barcelona (ES)
(72) Inventor: DUELO RIU, Juan José, 08017 BARCELONA (ES); DE LECEA FLORES DE LEMUS, Carlos, 08023 BARCELONA (ES); TINTORÉ GAZULLA, Maria, 08021 BARCELONA (ES); CUÑÉ CASTELLANA, Jordi, 08191 RUBÍ (ES); CARRERA MARCOLIN, Lydia, 08005 BARCELONA (ES); MARTÍ MELERO, Laia, 08005 BARCELONA (ES); PORTELA, Kristina Marianna, 08006 BARCELONA (ES); MARQUES LOPEZ-TEIJON, Borja, 08006 BARCELONA (ES); GARCIA-FAURA CIRERA, Alex, 08037 BARCELONA (ES)
(74) Representative: Ponti & Partners, S.L.P

(57) **Abstract**

The present invention relates to diamine oxidase (DAO) for use in the treatment of infertility.

## Description

### Field of the invention

The present invention relates to diamine oxidase (DAO) for use in the treatment of infertility in a human being, preferably a woman.

### Background

According to the World Health Organization (www.who.int), infertility is a global health issue affecting millions of people of reproductive age worldwide. Available data suggests that globally one in six people experience infertility in their lifetime. Infertility is a disease of the male or female reproductive system defined by the failure to achieve a pregnancy after 12 months or more of regular unprotected sexual intercourse. Primary infertility is the inability to have any pregnancy, while secondary infertility is the inability to have a pregnancy after previously successful conception.

Infertility may occur due to male factors, female factors, a combination of male and female factors or may be unexplained. Female infertility is often due to problems with ovulation that may be caused by ovulation disorders like polycystic ovary syndrome (PCOS), primary ovarian insufficiency (POI), or hyperprolactinemia. Female infertility can also be caused by uterine or cervical abnormalities, fallopian tube damage, uterine fibroids, endometriosis, early menopause, pelvic scar tissue, and even cancer treatment or severe psychological distress. Male infertility is most often caused by testicles that aren't working properly. Varicocele is a condition where the veins on a man's testicles are too large, which causes them to heat up, which affects sperm count and shape. The quality of sperm can also be affected by health conditions like diabetes, genetic defects, and undescended testicles. If sperm isn't delivered properly because of premature ejaculation or structural problems, this can also affect fertility. For both women and men, however, lifestyle factors such as smoking, excessive alcohol intake and obesity have been associated with higher chances of infertility.

In addition, infertility complications, like miscarriages, can negatively affect a person's overall health and quality of life. Many couples who want to start a family and are unable to conceive will experience psychological and interpersonal distress that could negatively impact their quality of life.

Despite the magnitude of the issue, solutions for the prevention, diagnosis and treatment of infertility - including assisted reproductive technology such as *in vitro* fertilization (IVF) - remain underfunded and inaccessible to many due to high costs, social stigma and limited availability.

At present, in most countries, fertility treatments are largely funded out of pocket - often resulting in devastating financial costs. People in the poorest countries spend a greater proportion of their income on fertility care compared to people in wealthier countries. High costs frequently prevent people from accessing infertility treatments or alternatively, can catapult them into poverty as a consequence of seeking care.

Treatments for infertility can range from medications to embryo implantation through assisted reproductive technology (ART). There are treatments that are specifically for men or for women and some that involve both partners. In 85% to 90% of cases, infertility is treated with conventional medical therapies, such as medication or surgery.

The most common medications used to treat female infertility help stimulate ovulation. Examples of these types of medications include: Clomiphene or Clomiphene Citrate, Letrozole, Gonadotropins or Human Chorionic Gonadotropin (hCG) and Bromocriptine or Cabergoline.

Treatments for male infertility may be based on the underlying cause of the problem, or in the case of no identified problem, evidence-based treatments that improve fertility may be recommended. In addition to treatments including surgery to correct or repair anatomic abnormalities or damage to reproductive organs or use of medical procedures to deliver sperm to the woman, fertilization of the egg in a laboratory, medication can treat some issues that affect male fertility, including hormone imbalances and erectile dysfunction. See www.nichd.nih.gov/health/topics/infertility/conditioninfo/treatments.

Accordingly, the problem to be solved by the present invention is to find an alternative way for treating infertility without the need of surgery.

The present invention addresses this problem by employing DAO.

**Diamine oxidase** (DAO) is an enzyme encoded by the AOC1 gene (OMIM#104610), located on chromosome 7 (7q-34-36). It is a secretory protein stored in vesicular structures of plasma membrane. It is responsible for the degradation of extracellular histamine. In mammals, DAO is expressed mainly in the small intestine and is located in the intestinal villi. DAO encoding gene polymorphisms have been analyzed in depth and more than 50 non-synonymous single nucleotide polymorphisms (SNPs) that can alter its activity have been identified. The three more relevant SNPs affecting Caucasian individuals that lead to a reduction of its enzymatic activity are: c.47C>T (rs10156191), c.995C>T (rs1049742), c.1990C>G (rs1049793) (Ayuso P, Garcia-Martin E, Martinez C, Agúndez JA. Genetic variability of human diamine oxidase: occurrence of three nonsynonymous polymorphisms and study of their effect on serum enzyme activity. Pharmacogenet Genomics. 2007;17(9): 687-693. doi:10.1097/FPC.0b013e328012b8e4; Garcia-Martin E, Ayuso P, Martinez C, Agúndez JA. Improved analytical sensitivity reveals the occurrence of gender-related variability in diamine oxidase enzyme activity in healthy individuals. Clin Biochem. 2007;40(16-17):1339-1341. doi:10.1016/j.clinbiochem.2007.07.019). Ayuso et al., described the following frequencies for these AOC1 variants (95% confidence interval) among Spanish Caucasian individuals: rs10156191, 25.4% (20.16-30.58), rs1049742, 6.3% (3.42-9.26) and rs1049793, 30.6% (25.1-36.1). In addition, the SNP c.691G>T (rs2052129) causing a decrease in DAO transcriptional activity has been identified in the promoter region of the AOC1 gene (Garcia-Martin E, Ayuso P, Martinez C, Agúndez JA. Improved analytical sensitivity reveals the occurrence of gender-related variability in diamine oxidase enzyme activity in healthy individuals. Clin Biochem 2007, 40, 1339-1341. doi: 10.1016/j.clinbiochem.2007.07.019).

### Summary of the invention

The present invention relates to diamine oxidase (DAO) for use in the treatment of infertility in a human being, preferably a woman.

### Detailed description

The present invention relates to diamine oxidase (DAO) for use in the treatment of infertility in a human being, preferably a woman.

In a preferred embodiment, said woman has been subjected to an unsuccessful previous treatment of infertility by medical uses or methods of treatment (for example the use of drugs or dietary supplements or functional foods for treating infertility), or to a previous unsuccessful transfer of embryos or has at least tried for one year to get pregnant without success, or any combination thereof.

In another preferred embodiment, said human being, preferably a woman, has a genetic DAO deficiency. More preferably, said human being, preferably a woman, has at least one homozygotic SNP variant in the AOC1 gene. Even more preferably, said human being, preferably a woman, has at least one homozygotic SNP variant in the AOC1 gene selected from SNP variants rs2052129 and or rs1049793.

In the present invention, "DAO" is the abbreviation used to designate the enzyme diamine oxidase responsible for the catalysis of the oxidative deamination of biogenic amines, for example of the primary amine group of histamine to give imidazole acetaldehyde. DAO in the present invention may have different origin, e.g. a non-plant origin, plant origin or biotechnological origin. "Non-plant origin" means any DAO that is not obtained from plants but from animal organisms or other non-plant organisms. Thus, this definition includes all DAO isolated from living creatures that are not plants. "Plant origin" means any DAO obtained from plant organisms. "Biotechnological origin" means any DAO prepared from recombinant cell cultures or in non-plant organisms of any type after isolating the DNA for DAO.

In this disclosure and in the claims, terms such as "comprises," "comprising," "containing" and "having" are open-ended terms and can mean "includes," "including," and the like; while terms like "consisting of" or "consists of" refer to the mentioned elements after these terms and others which are not mentioned are excluded.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include "and" unless the context clearly indicate otherwise.

It should be noted that the term "approximately" or "about" applied to the values used earlier and later in this document includes a margin of error of ± 5 %, such as, for example, ± 4 %, ± 3 %, ± 2 %, ± 1 %.

As used herein, the term "treat" or "treatment" or "treating" or grammatically related variants thereof means the amelioration, even temporarily, encompassing but not requiring the complete abolishment of a pathological state. In the context of the present invention, treatment of infertility by the medical uses and methods of treatment disclosed herein means achieving a pregnancy following a regimen of treatment as specified herein, where previous attempts to achieve pregnancy without applying such medical uses and methods of treatment have failed to result in the desired pregnancy.

In another embodiment, said diamine oxidase (DAO) is combined with at least one another active ingredient. Said at least one another active ingredient is an estrogen-receptor modifier such as clomiphene citrate, aromatase inhibitors such as letrozole, folic acid, tetrahydrofolate, D-chiro-inositol, myo-inositol, melatonin, vitamin D, vitamin B complex and vitamin B12, vitamin C, vitamin E, selenium, zinc, omega-3 poly-unsaturated oils, DHA, Q10 coenzyme, probiotics or postbiotics, β-carotene, cryptoxanthin, lycopene, N-acetylcysteine, L-arginine, carnitine, calcium, iodine, iron, magnesium, choline, biotin.

In a preferred embodiment, said diamine oxidase is included in a pharmaceutical composition. In a further preferred embodiment, said pharmaceutical composition further comprises a pharmaceutically acceptable vehicle, adjuvant, diluent or excipient, and optionally at least another active ingredient. Said pharmaceutically acceptable vehicle, adjuvant, diluent or excipient can be any of preserving agents, fillers, disintegrating agents, humidifying agents, emulsifying agents, suspending agents, solvents, dispersion media, coatings, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Said at least another active ingredient can be any of those mentioned above.

In another preferred embodiment, said treatment comprises topically, intravaginal, intra-urethral or orally administering said diamine oxidase or the pharmaceutical composition comprising thereof.

In one embodiment, the DAO or the pharmaceutical composition comprising thereof is administered topically. The dosage form may be selected from liquid in spray, cream, lotion, ointment, and liposomes.

In another embodiment, the DAO or the pharmaceutical composition comprising thereof is administered orally. The dosage form may be selected from a tablet, capsule, powder, microcapsule, minicapsule, nanocapsule, microemulsion, sachet, liposome, and drink. In another preferred embodiment, said dosage forms may have gastric protection for protecting the DAO from gastric acidity. The enteric coating layer that coats the various forms rapidly disintegrates or dissolves in a neutral or alkaline medium. The enteric coating layers may contain pharmaceutically acceptable plasticisers to obtain the desired mechanical properties of flexibility and hardness. These plasticisers can be, for example, triacetin, citric acid esters, phthalic acid esters, cetyl alcohol, polyethylene glycols, polysorbates or other plasticisers.

The presence of the gastric protection would depend on the dosage form. For example, for topical administration this gastric protection is not necessary. Accordingly, the present invention encompasses dosage forms with and without gastric protection.

The above mentioned dosage forms may also contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavouring agent such as peppermint, or methyl salicylate. In addition, dosage unit forms can contain various other materials that modify the physical form of the dosage unit, for example, coatings of sugar, shellac, or enteric agents. Other oral dosage forms like syrup or elixir may contain sweetening agents, preservatives, dyes, colourings, and flavourings. In addition, the active compounds may be incorporated into fast dissolve, modified-release or sustained-release preparations and formulations, and wherein such sustained-release formulations are preferably bimodal.

DAO or the compositions according to the present invention are capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition.

DAO can also be preferably mixed with pharmaceutically acceptable substances such as salts of phosphoric acid and sodium, potassium, calcium, magnesium and aluminium, carbonic acid, citric acid or other suitably weak organic and inorganic acids; a co-precipitate of aluminium hydroxide/sodium bicarbonate; substances normally used in anti-acid preparations such as hydroxides of aluminium, calcium and magnesium; magnesium oxide or compound substances such as Al₂O₃.6MgO.CO₂.12H₂O, (Mg₆Al₂(OH)₁₆CO₃.4H₂O, MgO.Al₂O₃.2SiO₂.nH₂O or similar compounds; pH buffering substances such as tris(hydroxymethyl)aminomethane, basic amino acids and their salts or other pharmaceutically acceptable pH buffering substances.

In another embodiment, the DAO or the pharmaceutical composition comprising thereof is administered intravaginally. In an embodiment, administering intravaginally is one or more of contacting the vagina, cervix, uterus, or any combination thereof of the female with an insert comprising DAO or the pharmaceutical composition comprising thereof, contacting the vagina, cervix, uterus, or any combination thereof of the female with a catheter comprising DAO or the pharmaceutical composition comprising thereof.

In a preferred embodiment, the insert is in the form of a vaginal suppository, pessary, tampon, sponge, or ring. In another preferred embodiment, the insert further comprises a polymer matrix, wherein preferably said polymer matrix comprises a crosslinked reaction product of a diisocyanate, a triol, and a polyethylene glycol. In another preferred embodiment, the insert further comprises a retrieval tape.

In a preferred embodiment, the catheter further comprises a DAO-porous surface adapted to contact the vagina.

In a preferred embodiment, the pharmaceutically composition comprising DAO for intravaginal administration further comprises a pharmaceutically acceptable carrier, and can be in the form of a cream, a gel, a capsule, a lotion, or a combination of two or more thereof.

In another embodiment, the DAO or the pharmaceutical composition comprising thereof is administered intra-urethrally. For such purpose, the composition can be in the form of a fluid or semi-fluid solution, suspension, dispersion, ointment, paste, cream, or gel which may also include many other excipients, including penetration enhancers, and buffering agents, as well as antimicrobial agents or preservatives for instance. All of these can be easily introduced into the urethra from a flexible tube, squeeze bottle, pump or aerosol spray in single or multiple dose administrations. The composition may also be contained in a rapidly releasing suppository which melts and is absorbed at room temperature. WO 91/16021 provides for devices to insert a suppository as well as coating the exterior of the urethral suppositor, also disclosed are devices for delivery of ointments, pastes, etc into the urethra. Such devices and coatings would also be applicable to the instant invention herein.

In another preferred embodiment, said diamine oxidase is included in a functional food or a dietary supplement (for example, refreshment, foodstuffs, chewing gums, sweets or candy), alone or optionally combined with at least one another active ingredient as mentioned above. In other words, said diamine oxidase is included in food matrices providing them with the enzymatic activity (functional food) and helping in reducing their content in biogenic amines. Diamine oxidase could be incorporated in such food matrices during the process of elaboration (processing aid) or as an ingredient declared in the composition of the finished product. Said diamine oxidase included in a functional food or a dietary supplement can be present with any media or agent/compound compatible with the diamine oxidase and the optional at least one another active ingredient. Examples of such media or agent/compound are preserving agents, fillers, disintegrating agents, wetting agents, emulsifying agents, suspending agents, solvents, dispersion media, coatings, isotonic and absorption delaying agents and the like. Said functional food or dietary supplement is preferably orally administered.

"Alone" is meant to be as the unique active ingredient but including any acceptable vehicle, adjuvant, diluent or excipient suitable for such functional food or dietary supplement.

In another embodiment, the pharmaceutical composition, the dietary supplement or the functional food comprising DAO is administered before or with meals once per day.

In another preferred embodiment, said diamine oxidase or the pharmaceutical composition comprising thereof is a cosmetical or medical device or is included in powder, semi-solid, semi-liquid or liquid form in a delivery system which is a medical device which facilitates the administration of the enzyme. Said diamine oxidase is present alone or optionally combined with at least one another active ingredient as mentioned above. Said diamine oxidase as a cosmetical or medical device or included in the cosmetical or medical device can be present with any media or agent/compound compatible with the diamine oxidase and the optional at least one another active ingredient. Examples of such media or agent/compound are preserving agents, fillers, disintegrating agents, wetting agents, emulsifying agents, suspending agents, solvents, dispersion media, coatings, isotonic and absorption delaying agents and the like.

The present disclosure also relates to a method of treating infertility, according to the embodiments included herein, comprising the step of administering a therapeutically effective amount or dose of DAO according to the embodiments included herein. The terms "therapeutically effective dose" and "therapeutically effective amount" are used to refer to an amount of the subject compound that results in a positive effect in the context of the present invention. A therapeutically effective amount, as well as a therapeutically effective frequency of administration, can be determined by methods known in the art and are within the skill of the attending physician or specialist.

In another preferred embodiment, the administration of the diamine oxidase according to any of the embodiments included herein is continued during pregnancy.

It is noted that any of the embodiments disclosed herein can be taken alone or combined with any other embodiment disclosed herein unless the context specifies otherwise. In other words, for example, a preferred option of a defined feature can be combined with a more or less preferred option of another feature.

The invention will be now illustrated with the following examples, which do not intend to limit its scope.

### EXAMPLES

### Example 1: Study to determine the prevalence of genetic DAO deficiency in infertile women attending to fertility clinics (PROTOCOL)

*Promotor identification*: DR Healthcare S.L.U.

*Center involved in the clinical trial*: Institut Marquès

*Ethical Committee:* All the materials have been reviewed by CEIC Hospital Clinic de Barcelona (Barcelona)

### Objective:

The aim of this study is to evaluate the prevalence of genetic DAO deficiency in infertile women attending to fertility clinics

### Main variables:

Number and percentage of women with DAO deficiency

### Study Design:

Monocentric, prospective clinical study to evaluate the prevalence of genetic DAO deficiency in infertile women.

### Selection of participants:

Adult women with fertility difficulties and attending to infertile clinic.

### Inclusion criteria:

- Women attending a fertility clinic due to fertility issues.
- Women with at least one year trying to get pregnant without success.

### Exclusion criteria:

- Women with exactly this condition: Older than 38 years old + in IVF (in vitro fertilization) process + to be implanted with their own eggs + without PGT (Pre-implantation Genetic Testing).
- Male partner with severe oligo or astenozoospermia.
- Pathological sperm FISH test or Meiosis.
- BMI (Body Mass Index) > 40
- Moderate or severe adenomyosis
- Patients who are taking DAO
- Karyotype altered without PGT

### Treatment and study calendar:

The investigator will recruit the patients as they come to consultation and meet the inclusion and exclusion criteria.

The expected duration for the recruitment of patients will be 4 months. After the patient recruitment, they will attend to 1 visit.

### Visit 1:

In this visit the patients will sign the informed consent form (if they agree to participate in the study) after reading the patients information sheet and receiving all the information about the study by investigators. The investigator will ensure that patients meet all the inclusion/exclusion criteria in order to be finally included in the study.

In order to characterize the population of patients, the following demographic parameters will be collected in this visit:
- Age
- Weight, height and BMI
- Infertility period and cause of infertility
- Ethnicity
- Information related to previous fertility
- Information related the current fertility treatment.

A sample of oral mucosa will be collected. The physician in charge of the medical assessment will be responsible to obtain an oral mucosa sample.

### Sample processing:

DNA sample for the genetic study will be obtained from an oral mucosa sample with a buccal swab. Samples will be stored at 4°C for up to 5 days, without compromising the quality of the DNA. Samples will be sent for analysis to Laboratorios Echevarne.

Samples can be stored for several days (maximum 1 week) at room temperature, preferably at 4°C until shipment. Shipment can be done at room temperature.

### Information about pregnancy:

Some information about the pregnancy will be collected.

### Statistical analysis:

The prevalence of DAO deficiency of genetic origin in the general population is described as 65% **https://doi.org/10.3390/genes16020141)**. The hypothesis of the present study is that the prevalence of DAO deficiency of genetic origin could be higher than that of the general population in patients with fertility problems, as occurs in patients with other pathologies.

In patients with a confirmed diagnosis of migraine, DAO deficiency has a prevalence of 87% (Izquierdo-Casas et al., 2018), and in a cohort of fibromyalgia patients, the prevalence of DAO deficiency of genetic origin was found to be 74.5% based on the four variants of the AOC1 gene (Okutan, et al. 2023). On another study of DAO deficiency, 78.8% of prevalence was found in a pediatric cohort of attention deficit hyperactivity disorder (ADHD) (Blasco-Fontecilla H, Bella-Fernández M, Wang P, Martin-Moratinos M, Li C. Prevalence and Clinical Picture of Diamine Oxidase Gene Variants in Children and Adolescents with Attention Deficit Hyperactivity Disorder: A Pilot Study. J Clin Med. 2024 Mar 14;13(6):1659. doi: 10.3390/jcm13061659. PMID: 38541885; PMCID: PMC10970994.).

As a working hypothesis, it is estimated that the prevalence of DAO deficiency of genetic origin in patients with fertility issues is equal to or greater than 65%.

A sample of 149 individuals is sufficient to estimate, with 95% confidence and an accuracy of +/- 8 percentage units, a population percentage that is expected to be around 60%. The percentage of necessary replacements is expected to be 3%.

Thus, a sample size of n = 150 volunteers is defined to establish the prevalence of DAO of genetic origin in patients with fertility issues.

A descriptive statistic of all the variables collected will be made.

The categorical variables will be presented in the form of lists of frequencies and proportions. For the quantitative variables, central tendency (mean and median) and dispersion (standard deviation, maximum and minimum values) will also be presented.

Primary endpoint: prevalence of DAO deficiency of genetic origin

The prevalence of DAO deficiency will be established based on the four variants of the AOC1 gene. The presence of at least one risk allele will be considered as genetic DAO deficiency.

Descriptive analysis and frequency comparisons are intended to be performed through Pearson's exact Chi-square test. Bilateral contrasts with a confidence level of 95% will be considered.

Association with pregnancy: The relationship between pregnancy and risk alleles will be performed using linear or logistic regression adjusting for age, calculating the odds ratio (OR) along with its 95% confidence (CI). To compare the intensity and frequency of the symptoms based on the risk alleles of the combined effect of the variants, the Kruskal-Wallis test and multiple comparisons will be used using the R statistical software and applying the applicable statistical corrections.

### Safety:

Sample collection poses no risk to study participants.

### Ethical and legal considerations:

This study will be performed in accordance with Declaration of Helsinki in its latest revised version (Fortaleza, 2013).

This study will be developed in accordance with what is established in this protocol and with the Law 14/2007 of 3^{rd} July on Biomedical Research.

Informed consent will be requested from patients prior to their inclusion in the study.

### Example 2. Results on screening of genetic DAO deficiency in implantation failure

### Objective:

The aim of this study is to estimate the prevalence of genetic DAO deficiency in infertile women attending fertility clinics. The hypothesis is that the prevalence of DAO deficiency of genetic origin could be higher than that of the general population in patients with fertility problems, as occurs in patients with other pathologies, such as lower urinary tract symptoms (LUTS), in which prevalence is 85.9%.

### Inclusion criteria:

The inclusion criteria are to be women attending the fertility clinic due to fertility issues and to have tried to get pregnant for at least one year without success.

### Genetic testing:

The genetic study of DAO deficiency is done by non-invasive techniques, by the analysis of a DNA sample from oral mucosa.

### Results:

**Table 1. Descriptive table of the sample population by pregnancy success**

| | | **NEGATIVE (N=57)** | **Positive (N=95)** | **Overall (N=152)** |
|---|---|---|---|---|
| **Global** | | | | |
| | ≥1 variant | 37 (64.9%) | 67 (70.5%) | 104 **(68.4%)** |
| | Normal | 20 (35.1%) | 28 (29.5%) | 48 (31.6%) |

| **x691g_t** | | | | |
|---|---|---|---|---|
| | Heterozygosis | 16 (28.1%) | 33 (34.7%) | 49 (32.2%) |
| | Homozygosis | 3 (5.3%) | 4 (4.2%) | 7 (4.6%) |
| | Normal | 38 (66.7%) | 58(61.1%) | 96 (63.2%) |

| **x47c_t_rs10156191** | | | | |
|---|---|---|---|---|
| | Heterozygosis | 18(31.6%) | 40(42.1%) | 58 (38.2%) |
| | Homozygosis | 3 (5.3%) | 4 (4.2%) | 7 (4.6%) |
| | Normal | 36 (63.2%) | 51 (53.7%) | 87 (57.2%) |

| **x995c_t_rs 1049742** | | | | |
|---|---|---|---|---|
| | heterozygosis | 3 (5.3%) | 8 (8.4%) | 11 (7.2%) |
| | homozygosis | 0 (0.0%) | 0 (0.0%) | 0 (0.0%) |
| | Normal | 54 (94.7%) | 87 (91.6%) | 141 (92.8%) |

| **x1990c_g_rs1049793** | | | | |
|---|---|---|---|---|
| | | **NEGATIVE (N=57)** | Positive **(N=95)** | **Overall (N=152)** |
| | Heterozygosis | 14 (24.6%) | 38 (40.0%) | 52 (34.2%) |
| | Homozygosis | 8 (14.0%) | 7 (7.4%) | 15 (9.9%) |
| | Normal | 35 (61.4%) | 50 (52.6%) | 85 (55.9%) |

| **Age** | | | | |
|---|---|---|---|---|
| | Mean (SD) | 39.9 (4.96) | 40.4 (5.59) | 40.2 (5.35) |
| | Median [Q1, Q3] | 40.0 [36.0, 43.0] | 41.0 [37.0, 44.0] | 41.0 [36.0, 44.0] |
| | Missing | 2 (3.5%) | 1 (1.1%) | 3 (2.0%) |

| **Height** | | | | |
|---|---|---|---|---|
| | Mean (SD) | 1.64 (0.0640) | 1.63 (0.0729) | 1.63 (0.0696) |
| | Median [Q1, Q3] | 1.64 [1.60, 1.68] | 1.63 [1.60, 1.69] | 1.63 [1.60, 1.68] |
| | Missing | 5 (8.8%) | 5 (5.3%) | 10 (6.6%) |

| **Weight** | | | | |
|---|---|---|---|---|
| | Mean (SD) | 65.0 (14.4) | 63.1 (11.2) | 63.8 (12.5) |
| | Median [Q1, Q3] | 61.5 [55.0, 70.5] | 60.0 [55.0, 68.0] | 60.0 [55.0, 69.0] |
| | Missing | 5 (8.8%) | 6 (6.3%) | 11 (7.2%) |

| **BMI** | | | | |
|---|---|---|---|---|
| | Mean (SD) | 24.2 (4.95) | 23.9 (4.24) | 24.0 (4.50) |
| | Median [Q1, Q3] | 22.1 [20.8, 27.4] | 22.8 [20.8, 25.4] | 22.6 [20.8, 26.1] |
| | Missing | 5 (8.8%) | 6 (6.3%) | 11 (7.2%) |

| Race | | | | |
|---|---|---|---|---|
| | | **NEGATIVE (N=57)** | **Positive (N=95)** | **Overall (N=152)** |
| | Arabic | 1 (1.8%) | 3 (3.2%) | 4 (2.7%) |
| | Caucasian | 48 (87.3%) | 89 (93.7%) | 137 (91.3%) |
| | Latin American | 3 (5.5%) | 2 (2.1%) | 5 (3.3%) |
| | Mixed | 2 (3.6%) | 1 (1.1%) | 3 (2.0%) |
| | Black | 1 (1.8%) | 0 (0%) | 1 (0.7%) |
| | Missing | 2 (3.5%) | 0 (0%) | 2 (1.3%) |

68.4% (104) of subjects with infertility have at least one mutation, either in heterozygosis or in homozygosis.

**Table 2. Pregnancy test outcomes by presence of homozygotic SNP variants in the AOC1 gene**

| | **NEGATIVE (N=57)** | **Positive (N=95)** | **Overall (N=152)** |
|---|---|---|---|
| **0 variants in homozygosis** | 45 **(33.88%)** | 84 **(65.12%)** | 129 (100%) |
| **≥1 variant in homozygosis** | 12 **(52.17%)** | 11 (47.83%) | 23 (100%) |

Descriptive results show that the majority of subjects without homozygotic variants obtain positive pregnancy test results (65.12%). On the contrary, among subjects with **at least one SNP in homozygosity,** the **majority** obtains **negative pregnancy test results** (52.17%).

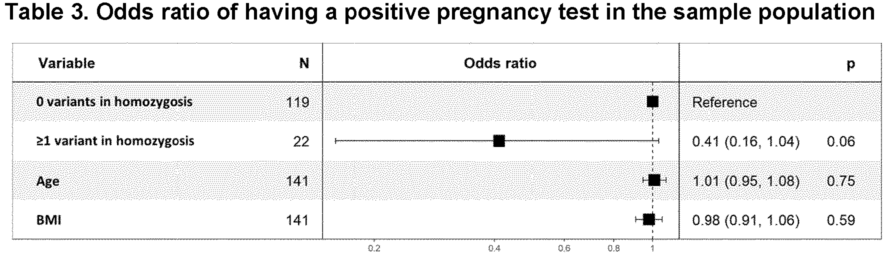

Subjects with at least one gene variant (SNP) in homozygosis have **59% lower odds (1** - 0.41) of having a positive pregnancy result, compared to the control (subjects with no variants in homozygosis).

Age and BMI do not influence the likelihood of becoming pregnant (p = 0.75 and 0.59, respectively).

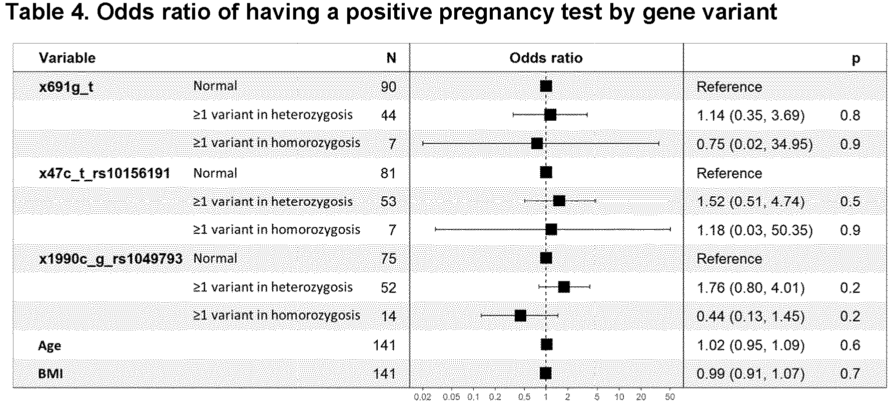

Although there is not enough statistical significance to establish an association, there is a trend for homozygotic subjects to have lower odds of having a positive pregnancy test. This effect can be clearly seen especially for SNP rs1049793, with 56% lower odds (1 - 0.44) of having a positive pregnancy test compared to subjects with no mutations.

### Example 3: Pilot study to determine the effect of DAO (Diamine Oxidase) supplementation in infertile women with DAO deficiency (PROTOCOL)

*Promotor identification:* DR Healthcare S.L.U.

*Center involved in the clinical trial:* Institut Marquès

*Ethical Committee:* All the materials have been reviewed by CEIC Hospital Clinic de Barcelona (Barcelona)

### Objective:

The aim of this study is to evaluate the influence of DAO supplementation in infertile women with previous unsuccessful embryo transfer and DAO deficiency.

The genetic study of DAO deficiency can be done by non-invasive techniques, by the analysis of a DNA sample from oral mucosa, which do not represent any inconvenience or side effect for the volunteers of the study, and supplementation with DAO enzyme is available.

### Main variables:

### Result of ß test

### Secondary variables:

- Successful pregnancy
- Miscarriage Rate

### Study Design:

Monocentric, prospective, randomized, double-blind and placebo-controlled clinical study to evaluate the influence of DAO supplementation in infertile women with DAO deficiency.

They will be randomized into two groups: experimental group and control group (Placebo). The experimental group will receive a daily dose of 25,2 mg of DAO, divided into 3 doses of 8,4 mg per day, taken before the main meals for two months. The control group will receive the same dose of placebo under the same posology for the same period of time.

The intervention group will be assigned to each patient according to the assigned code in order of inclusion to the study and that will serve for the randomization of the patients. The code lists will be previously prepared through an informatic program. The ratio of experimental and control group will be 1:1. The study is designed to make the assignment double-blinded; therefore, the randomization sequence will be hidden and only visible by the study monitor.

The Placebo and DAO capsules will be prepared in such a way that they are indistinguishable from each other. The participants in the study, as well as the technicians in contact with the patients, in charge of preparing questionnaires, distributing capsules, etc., will not be able to know which capsules correspond to each experimental group.

*Selection of participants:* Adult women with DAO deficiency and after an unsuccessful embryo transfer.

### Inclusion criteria:

- Women with DAO deficiency
- Women attending a fertility clinic due to fertility issues with previous unsuccessful results in embryo transfer (negative test ß or non-evolutionary pregnancy) .
- Women who will perform an embryo transfer in 2-3 months.

### Exclusion criteria:

- Women with exactly this condition: Older than 38 years old + in IVF (in vitro fertilization) process + to be implanted with their own eggs + without PGT (Pre-implantation Genetic Testing).
- Male partner with severe oligo or astenozoospermia.
- Pathological sperm FISH test or Meiosis.
- BMI (Body Mass Index) > 40
- Moderate or severe adenomyosis
- Patients who are taking DAO or intending to take it during the study
- Karyotype altered without PGT

### Treatment and study calendar:

The investigator will recruit the patients as they come to consultation and meet the inclusion and exclusion criteria. The assignment of the study treatment will be carried out randomly.

The expected duration for the recruitment of patients will be 12 moths. After the patient recruitment, they will attend to 1 visit and some information will be collect after the embryo transfer in visit for routine clinical practice of assisted reproduction treatment.

### Visit 1:

In this visit the patients will sign the informed consent form (if they agree to participate in the study) after reading the patients information sheet and receiving all the information about the study by investigators. The investigator will ensure that patients meet all the inclusion/exclusion criteria in order to be finally included in the study.

In order to characterize the population of patients, the following demographic parameters will be collected in this visit:
- Age
- Weight, height and BMI
- Infertility period and cause of infertility
- Ethnicity
- Information related to previous fertility
- Information related the current fertility treatment.

Patients will take one capsule of DAO supplementation (8,4 mg) or placebo three times per day before the main meals for 2 months.

### Follow-up:

After the DAO supplementation and the embryo transfer the information about the results of the fertility treatment will be collected in the visits for routine clinical practice.

### Information about pregnancy:

Some information about the pregnancy will be collected.

### Statistical analysis:

This is a pilot study to understand if the success rate of a fertilization cycle could be improved by DAO supplementation in infertile women with DAO deficiency. Considering that there is no previous evidence in which to base a sample size calculation, and that any improvement in the pregnancy rate with regards to the placebo group can be considered as clinically relevant, our estimation is based on the recruitment potential of the center. It is estimated that 50 women compliant with the inclusion criteria and undergoing a fertilization cycle can be recruited in 12 months. If the results of this study are promising, a new study could be envisaged with a bigger sample size, calculated upon the data made available by this study.

A descriptive statistic of all the variables collected will be made.

The categorical variables will be presented in the form of lists of frequencies and proportions. For the quantitative variables, central tendency (mean and median) and dispersion (standard deviation, maximum and minimum values) will also be presented.

Primary endpoint: Biochemical pregnancy success rate in women with prevalence of DAO deficiency of genetic origin supplemented with DAO or with placebo.

### Safety:

The product in study is commercialized and no adverse reactions or tolerability issues have been described until now, not even expected.

The product is an extract from pea sprout with a long history of human consumption. As such, it was not deemed to be a Novel Food by the European Food Safety Authority and its use and commercialization are allowed. It was launched commercially as Food for Special Medical Purposes for the dietary management of DAO deficiency in 2021 and since then it has been in the market without any reported tolerability issue or side effect.

### Ethical and legal considerations:

This study will be performed in accordance with Declaration of Helsinki in its latest revised version (Fortaleza, 2013).

This study will be developed in accordance with what is established in this protocol and with the Law 14/2007 of 3^{rd} July on Biomedical Research.

Informed consent will be requested from patients prior to their inclusion in the study.

### Example 4. Preliminary results on DAO supplementation in patients with implantation failure

### Justification and objective:

Whether women with histamine intolerance, low DAO activity or genetic DAO deficiency before getting pregnant might be associated with conception difficulty, complicated pregnancies or higher risk of abortion has not been investigated yet. The aim of this study is to estimate the prevalence of genetic DAO deficiency in infertile women attending fertility clinics. The pregnancy success rate of patients that first had an unsuccessful embryo transfer and try for the second time is around 40-50%. The hypothesis is that the prevalence of DAO deficiency of genetic origin could be higher than that of the general population in patients with fertility problems, as occurs in patients with other pathologies, such as lower urinary tract symptoms (LUTS), in which prevalence is 85.9%.

### Study Design:

A monocentric, prospective, randomized, double-blind and placebo-controlled clinical study is performed to evaluate the influence of DAO supplementation in infertile women with DAO deficiency.

### Inclusion criteria:

The inclusion criteria are:
- To be a woman with genetic DAO deficiency
- To attend the fertility clinic due to fertility issues with previous unsuccessful results in embryo transfer (negative ß test or non-evolutionary pregnancy).
- To plan to perform an embryo transfer in 2-3 months.

### Supplementation:

Volunteers will be randomized into one of two groups: experimental group and control group (Placebo). The experimental group will receive a daily oral dose of 25,2 mg of DAO, divided into 3 doses of 8,4 mg per day, taken before the main meals for 2 months. The control group will receive the same dose of placebo under the same posology for the same period of time.

### Results:

- Total count of supplemented volunteers with a pregnancy result from the 2^{nd} transfer: 2

| **Patient ID** | **Start supplementation** | **Date 2^{nd} transfer** | **Pregnancy result** |
|---|---|---|---|
| 964321 | 30/05 | 12/07 | + |
| 964516 | 31/05 | 23/09 | + |

- Clinical pregnancy rate: all patients achieved a confirmed pregnancy within 2 months of treatment.
- Safety: The supplement was well-tolerated, with no reported adverse events.

### Conclusions:

These preliminary data suggest that DAO is effective in improving pregnancy rates in women with infertility.

## Claims

1. Diamine oxidase (DAO) for use in the treatment of infertility in a human being.

2. The diamine oxidase for use according to claim 1, wherein said human being is a woman.

3. The diamine oxidase for use according to claim 2, wherein said woman has been subjected to an unsuccessful previous treatment of infertility by medical uses or methods of treatment, or to a previous unsuccessful transfer of embryos or has at least tried for one year to get pregnant without success or any combination thereof.

4. The diamine oxidase for use according to claim 2 or 3, wherein said woman has at least one homozygotic SNP variant in the AOC1 gene.

5. The diamine oxidase for use according to claim 4, wherein said at least one homozygotic SNP variant in the AOC1 gene is selected from SNP variants rs2052129 and or rs1049793.

6. The diamine oxidase for use according to any of claims 1 to 5, wherein said DAO is combined with at least one another active ingredient.

7. The diamine oxidase for use according to claim 6, wherein said at least one another active ingredient is clomiphene citrate, letrozole, folic acid, tetrahydrofolate, D-chiro-inositol, myo-inositol, melatonin, vitamin D, vitamin B complex, vitamin B12, vitamin C, vitamin E, selenium, zinc, omega-3 poly-unsaturated oils, DHA, Q10 coenzyme, probiotics or postbiotics, β-carotene, cryptoxanthin, lycopene, N-acetylcysteine, L-arginine, carnitine, calcium, iodine, iron, magnesium, choline, and biotin.

8. The diamine oxidase for use according to any of the preceding claims, wherein said diamine oxidase is included in a pharmaceutical composition.

9. The diamine oxidase for use according to claim 8, wherein said pharmaceutical composition further comprises a pharmaceutically acceptable vehicle, adjuvant, diluent or excipient, and optionally at least another active ingredient.

10. The diamine oxidase for use according to any of the preceding claims, wherein said treatment comprises topically, intravaginal, intra-urethral or orally administering said diamine oxidase or the pharmaceutical composition comprising thereof.

11. The diamine oxidase for use according to claim 10, wherein said prevention or treatment comprises orally administering said diamine oxidase or the pharmaceutical composition comprising thereof.

12. The diamine oxidase for use according to claim 11, wherein said diamine oxidase or the pharmaceutical composition comprising thereof is administered orally in a dosage form selected from a tablet, capsule, microcapsule, minicapsule, nanocapsule, microemulsion, sachet, liposome and drink.

13. The diamine oxidase for use according to any of the claims 1-7, wherein said diamine oxidase is included in a functional food or a dietary supplement.

14. The diamine oxidase for use according to any of the claims 1-7, wherein said diamine oxidase is a cosmetic or a medical device or is included in powder, semi-solid, semi-liquid or liquid form in a delivery system which is a medical device.

15. The diamine oxidase for use according to any of claims 2 to 14, wherein said treatment continues during pregnancy.
